# EUROPEAN PATENT APPLICATION

(11) **EP 1 647 295 A1**
(43) Date of publication of application: **19.04.2006**
(21) Application number: 05021594.6
(22) Date of filing: 04.10.2005
(51) Int. Cl.: A61M 16/04

(54) **Device for retrograde nasotracheal intubation**

(30) Priority: 13.10.2004 IT MO20040272
(71) Applicant: H.S.-Hospital Service-Spa, 00040 Pomezia (IT)
(72) Inventor: Dauri, Mario, 00187 Roma (IT); Tosoratti, Nevio, 00151 Roma (IT)
(74) Representative: Crugnola, Pietro

(57) **Abstract**

A device (1) for retrograde nasotracheal intubation of a patient, comprising:
- a flexible endotracheal tube (1) that at its proximal end has a removable terminal portion (4) provided with an axial hole (5), and, at its distal end, an inflatable seal cuff (C) ;
- inflating means (7, 7a) of said seal cuff (C), associated with said endotracheal tube (1) and comprising an inflating tube (7) obtained in the thickness of the wall (10) of said endotracheal tube (1).

## Description

The present invention relates to a device for nasotracheal intubation used for mechanical ventilation in anaesthesia and intensive therapy.

In the prior art it is known that nasotracheal intubation is preferable to orotracheal intubation both in intensive therapy - inasmuch as nasotracheal intubation causes less discomfort to the patient and enables the endotracheal tube (TET) that is used for mechanical ventilation of the patient to stay in place more securely - and in anaesthesia, in particular types of operations, for example oral and maxillofacial surgery, in order to prevent the encumbrance of the tube during surgery.

Nasotracheal intubation can be conducted in an anterograde manner by inserting the TET through a nostril of the patient to advance it therefrom into the larynx and the trachea.

This technique has the drawback that the TET, whilst traversing the nasal cavities, the pharynx and the larynx, before being positioned in the patient's trachea, may easily cause lesions to the mucous, with consequent bleeding, possible avulsion of structures, and the risk of the passage of potentially septic matter into the air passages. Furthermore, there is also the risk that the cuff of the TET may break, which is used to ensure the seal of the tube during positive-pressure ventilation.

In order to reduce the risks specified above, recourse can be made to retrograde nasotracheal intubation, in which the TET is inserted orally and after insertion the proximal part of the tube is made to pass in a retrograde manner through the nasal cavities of the patient in such a way as to exit from a nostril.

The use of a standard-type TET for a retrograde nasotracheal intubation nevertheless brings with it the risk of lesions or of damage to the tube inasmuch as normal endotracheal tubes are not specifically designed for this type of insertion.

From Italian patent application RM2001A000536 a device is known that is suitable for retrograde nasotracheal intubation that comprises a flexible TET, that at its proximal end has a tapered removable portion provided with an axial hole and at its distal end a cuff for ensuring the seal of the TET during positive-pressure ventilation. The cuff is inflated by an inflating tube the terminal portion of which runs outside the TET to then withdraw inside the latter through a suitable hole in the side walls just below the detachment line of the removable end of the TET. The device furthermore comprises a connector for connecting the TET, after removal of its removable end, to a manual or mechanical ventilation system, and a traction catheter or wire the distal end of which has secure fixing means at the proximal removable end of the TET, within which it is inserted through the respective axial hole, so as to enable rapid and firm traction to recover the proximal end of the TET through the patient's nose.

The device disclosed in RM2001A000536 has the drawback that during the operation of intubation of the patient, the end portion of the inflating tube may easily remain jammed and oppose resistance during the TET recovery operation through the nose of the patient, with the risk of lesions to the patient and complications in the intubation operation; furthermore, the inevitable proximity of the detachment line of the removable tapered head of the TET and of the point in which the inflating tube of the seal cuff of the TET withdraws from the outside to the inside of the TET increases the probability of accidental damage to the inflating tube during the act of removal of the removable end of the TET.
The present invention aims to remedy the above drawbacks and to minimise the lateral dimensions of the TET.

According to the present invention a device is provided for retrograde nasotracheal intubation comprising:
- a flexible endotracheal tube that at its proximal end has a removable terminal portion, and, at its distal end, an inflatable seal cuff, said terminal portion being provided with an axial hole;
- inflating means of said seal cuff (C), associated with said endotracheal tube, characterised in that said inflating means comprises an inflating tube obtained in the thickness of the wall of said endotracheal tube.

The fact that the inflating tube of the cuff is obtained in the thickness of the wall of the endotracheal tube minimises the overall lateral dimensions of the tube.

According to a preferred version of the present invention the side wall of the endotracheal tube is provided with a groove intended to house an extractable end of the inflating tube of the seal cuff of the TET, said groove being shaped in such a way as to retain within itself said extractable end and to enable the extraction thereof.

Owing to this feature of the invention the risk is eliminated that the extractable end of the inflating tube may constitute either an obstacle to the recovery of the proximal end of the endotracheal tube through the nasal cavities of the patient or constitute a potential source of lesions.

Further advantages and features of the present invention will be clear from the following disclosure that is provided purely by way of non-limitative example with reference to the attached drawings in which:
Fig. 1 is a schematic view of a device for retrograde nasotracheal intubation according to the invention;
Figs 2, 3, 4 and 5 illustrate possible securely fixable traction means for recovering the proximal end of the TET from the nasal cavity; in particular, Fig. 2 illustrates a traction wire that enables fixing by means of a particular conformation of its distal end, shown in Fig. 4 in the initial configuration and in Fig. 5 in the final expanded configuration; an alternative traction means consisting of a Fogarty catheter with inflatable cuff at the distal end is illustrated in Fig. 3;
Fig. 6 illustrates a standard connector for connecting the device according to the invention to an external ventilation system;
Figs 7 and 8 are enlarged details of the device in Fig. 1 that illustrate the detail of the inflating tube of the seal cuff of the TET located inside the wall of the TET, respectively in rest and operating configuration conditions with the flexible terminal portion of said tube extracted from its seat to enable the connection to an external line for inflating the seal cuff of the TET;
Figs 9 and 10 are further enlarged particulars of the device in Fig. 1 that illustrate further details regarding the inflating tube inside the TET, with particular reference to the groove within which this tube is housed, respectively in a frontal view and in a longitudinal view;
Fig. 11 illustrates the connection system between the external inflating line and the flexible terminal portion of the inflating tube inside the wall of the TET by means of a suitable connector, two possible embodiments of which are shown;
Fig. 11a illustrates the external inflating line;
Figs 12 and 13 show an enlarged detail of the device according to the invention to illustrate the seal cuff respectively before and after inflating;
Figs 14 and 15 are greatly enlarged details of Figs 12 and 13 respectively that illustrate the connection between the inflating tube obtained inside the wall of the TET and the seal cuff.

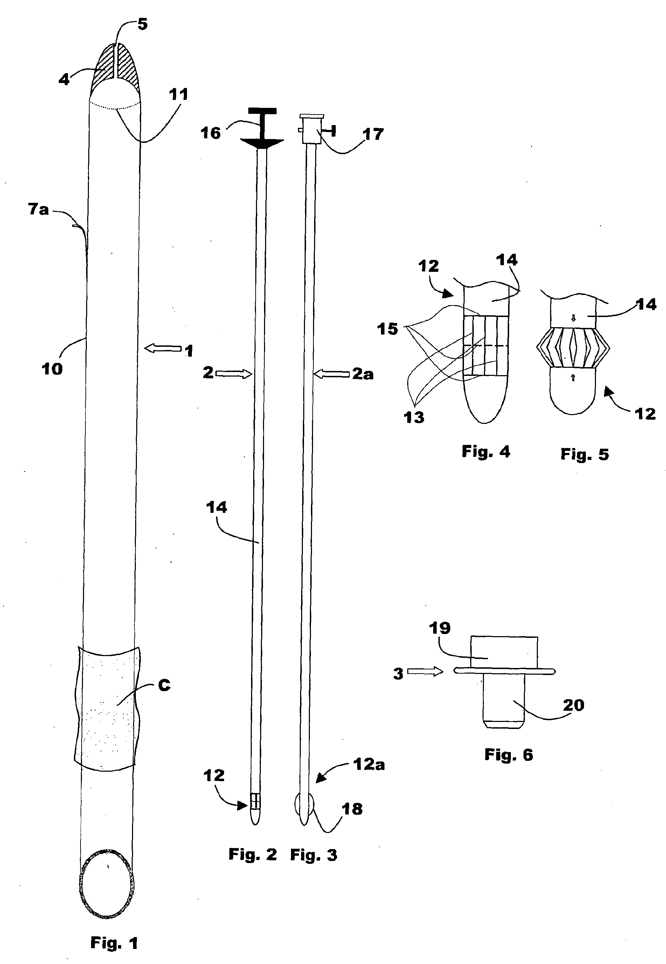

The device according to the invention, schematized in Fig. 1, comprises a flexible endotracheal tube (TET) 1 at the distal end of which an inflatable cuff C is provided the function of which is to ensure the seal of the TET 1 after the latter has been inserted into the patient's trachea.

The proximal end of the flexible tube 1 comprises a removable terminal portion 4, for example nose-shaped, in which an axial hole 5 is made through which it is possible to insert traction means 2, 2a, for example like those illustrated in Figs 2 and 3, to recover through the nasal cavities of the patient the proximal end of the TET 1 after the distal end has been positioned in the trachea. The traction means may consist of a catheter 2a or of a traction wire 2.

The traction wire 2 (Fig. 2), of known type, is provided at its distal end with fixing means 12 consisting of a portion of wire divided into a plurality of segments 13 in each of which weakening lines 15 are obtained that enable the segments to withdraw to the outside (Fig. 5) to immobilise the traction wire 2 against the base wall of the proximal end of the tube 1.

The catheter 2a (Fig. 3) is a catheter of the Fogarty type, which is also known, in which the fixing means 12a consists of an inflatable cuff 18.

The nose-shaped terminal portion 4 of the proximal end of the TET 1, after the tube has been positioned in the trachea of the patient and said proximal end has been recovered through the nasal cavities, is removed by using a scalpel, or other instrument with similar function, by means of a cut along the appropriate detachment line 11 located at the base of said terminal portion 4.

Subsequently, the TET 1 is connected to a device for ventilation of the patient by a connector 3 (Fig. 6). This connector comprises a first portion 19, with a cylindrical shape, suitable for being connected to said ventilation device, and a second portion 20, also with a cylindrical shape, intended to be inserted into the proximal end of the TET 1 - open as said, at this stage of the intubation procedure-. The external diameter of the second portion 20 of the connector 3 element is selected in such a way that the coupling between said second portion 20 and the internal wall of the TET 1 occurs with interference to ensure a seal when the ventilation device blows air into the TET 1; in other words, the external diameter of the second portion 20 of the connector element 3 has been selected slightly greater than the internal diameter of the TET 1.

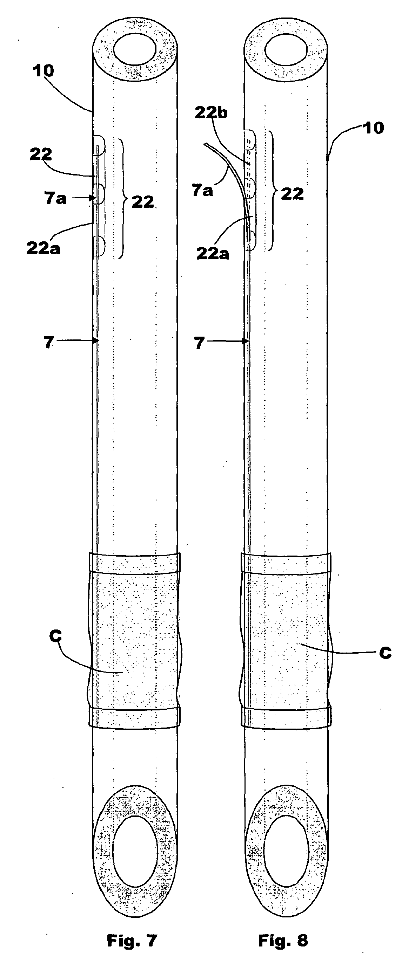

In the side wall 10 of the TET 1, rather below the removable terminal portion 4, a groove 22 (Figs 7, 8, 9, 10) is made, suitable for housing until the end of the operation of intubation of the patient (Fig. 7) a flexible end 7a of an inflating tube 7 of the cuff C, obtained inside the wall 10 of the TET. Said groove 22 comprises a first portion 22a opened laterally and therefore accessible from the outside of the TET 1, and a second portion 22b closed at the top end and laterally. The particular conformation of the groove 22 on one side does not increase the total lateral dimensions of the TET, thus preventing the degree of invasiveness being increased and on the other hand ensures that the flexible end 7a of the inflating tube 7 does not protrude from the wall 10 during the insertion of the TET 1 and during the recovery of its proximal end through the nasal cavities of the patient, nevertheless enabling its subsequent immediate extraction through the laterally open portion of the groove 22, as shown in Fig. 8, for example by means of pliers or another similar instrument, for the purpose of the subsequent connection to the external inflating line 6.

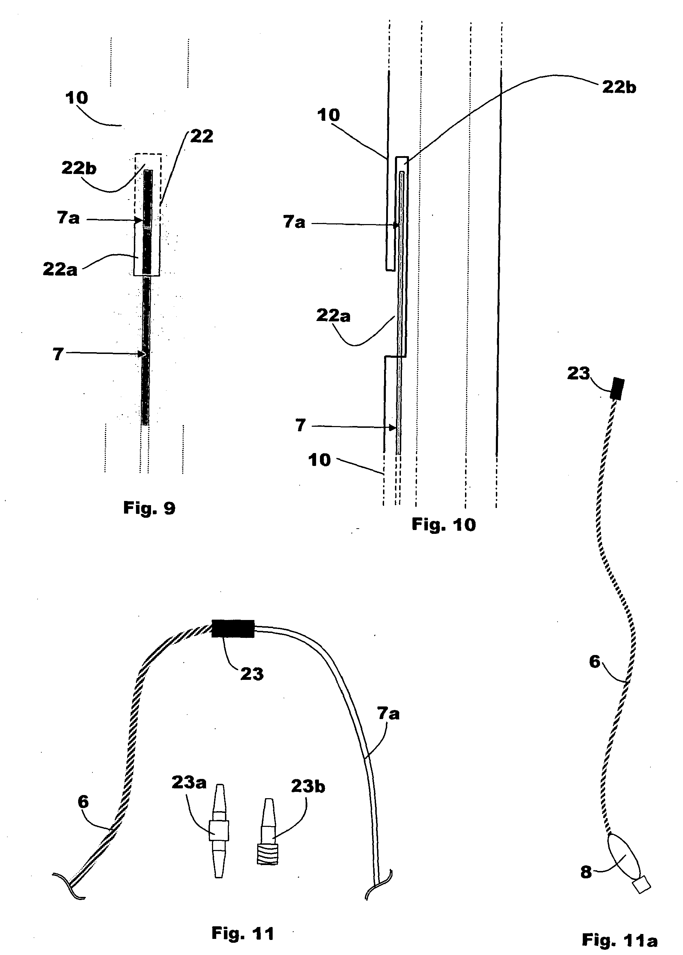

Said connection occurs through an appropriate connector 23, for example one of the two types illustrated in Fig. 11: a double-tapered connector 23a in metal or plastic material or a connector 23b provided with a taper at one end and with a screwable or bayonet connecting element at the other end. This connector 23 is normally already inserted into the distal end of the external inflating line 6 and has to be connected to the free end 7a of the inflating tube 7 inside the wall 10 of the head TET 1 after its extraction from the groove 22, at the end of the operations of intubation of the patient and of recovery of the TET 1 outside the nasal cavity.

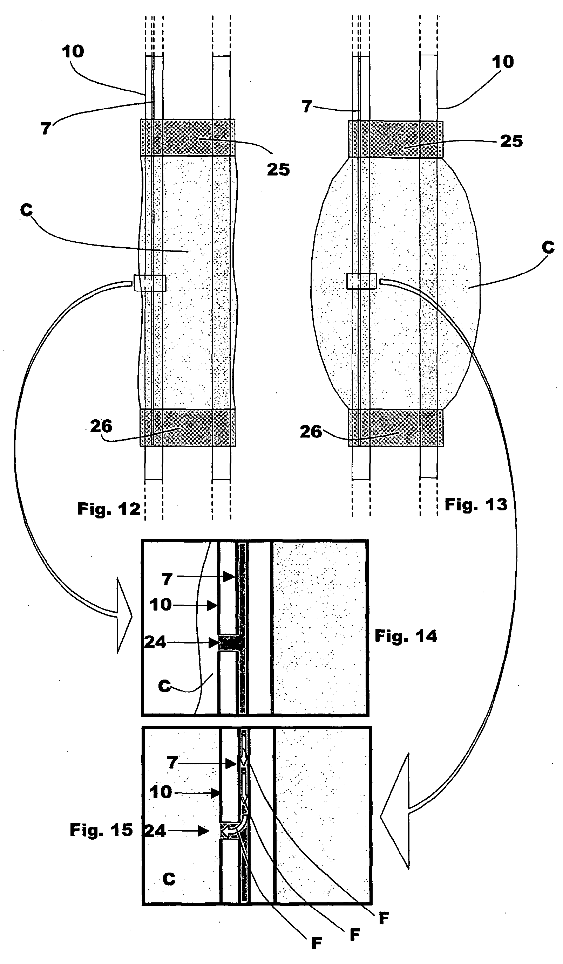

Once this connection has been made, inflating the seal cuff C is possible, that may be made due to the presence of an opening 24 (Figs 14 and 15) through the side wall 10 of the TET 1 that enables the flow of air F from the inflating tube 7, obtained inside the wall 10, to the seal cuff C. This cuff C consists of a sleeve in elastic material applied coaxially on the lateral wall 10 of the TET 1, and fixed to it at its two ends 25, 26 in such a way as to ensure an air seal.

The external inflating line consists of a flexible inflating tube 6 and in its proximal portion it is provided with a monitoring ball 8 for the control of pressure inside the seal cuff 7 of the TET in the patient's trachea.

The TET 1 can be provided on its lateral surface with position indicators and be provided with an opaque radio line along its entire length or in some of its parts or with other means useful for correct display and evaluation of the positioning of the device by radiographic means.

In the practical embodiment, the materials, dimensions and constructional details may be different from those indicated but be technically equivalent thereto without thereby falling outside the scope of the present invention.

## Claims

1. Device (1) for retrograde nasotracheal intubation of a patient comprising:
- a flexible endotracheal tube (1) that at its proximal end has a removable terminal portion (4) provided with an axial hole (5), and, at its distal end, an inflatable seal cuff (C);
- inflating means (7, 7a) of said seal cuff (C), associated with said endotracheal tube (1),
**characterised in that** said inflating means comprises an inflating tube (7) obtained in the thickness of the wall (10) of said endotracheal tube (1).

2. Device according to claim 1, wherein an end (7a) of said inflating tube (7) is extractable outside said wall (10).

3. Device according to claim 2, wherein said wall (10) is provided with a groove (22) intended to house said end (7a) of said inflating tube (7), said groove (22) being shaped in such a way as to retain said end (7a) within itself and to enable the extraction thereof.

4. Device according to claim 3, wherein said groove (22) comprises a first portion (22a) opened sideways then accessible from the outside of the endotracheal tube (1), and a second portion (22b) closed at the top end and laterally.

5. Device according to any preceding claim, wherein said wall (10) is provided with a transverse hole (24) communicating at an end with said cuff (C) and at the other end with said inflating tube (7).

6. Device according to any preceding claim, wherein said cuff (C) consists of a sleeve in elastic material applied coaxially to said wall (10) and sealingly fixed to it at one of its two ends (25, 26).

7. Device according to any preceding claim, furthermore comprising connecting means (3) to connect said endotracheal tube to a ventilation device, said connecting means being sealingly couplable with said endotracheal tube (1).

8. Device according to claim 7, wherein said seal is made by mechanical interference between said connecting means (3) and said endotracheal tube (1).

9. Device according to claim 8, wherein said connecting means (3) comprises a first part (19), cylindrical in shape, intended to be connected to said ventilation device and a second part (20), also cylindrical in shape, intended to be connected to the endotracheal tube (1), the external diameter of said second part (20) being selected in such a way as to create said mechanical interference.

10. Device according to any preceding claim, furthermore comprising further connecting means (23, 23a, 23b) suitable for connecting said inflating tube (7) to an external inflating line (6) connected to a suitable external inflating device.

11. Device according to claim 10, wherein said further connecting means (23, 23a, 23b) comprises a double-tapered connector (23a).

12. Device according to claim 10, wherein said further connecting means (23, 23a, 23b) comprises a connector (23b) having a first taper-shaped end and a second threaded end or end provided with a bayonet-fitting means.

13. Device according to any preceding claim, wherein said endotracheal tube (1) is provided, on its external surface, with position-indicating means.

14. Device according to claim 13, wherein said position-indicating means comprises an opaque radio line arranged longitudinally along the external surface of said endotracheal tube (1) along its entire length or in some parts thereof.
